# EUROPÄISCHE PATENTANMELDUNG

(11) **EP 0 786 423 A1**
(43) Veröffentlichungstag der Anmeldung: **30.07.1997**
(21) Anmeldenummer: 96100934.7
(22) Anmeldetag: 23.01.1996
(51) Int. Cl.: B65F 1/10, B65F 1/14

(54) **Abfallschleuse**

(71) Anmelder: InnoRatio Aktiengesellschaft für innovative umwelttechnische Systeme, 83022 Rosenheim (DE)
(72) Erfinder: Kardum, Iwe, D-84524 Neuötting (DE); Ebner, Rudolf, D-84431 Rattenkirchen (DE)
(74) Vertreter: Müller-Boré & Partner Patentanwälte

(57) **Zusammenfassung**

Die vorliegende Erfindung betrifft eine Abfallschleuse (10) für Mülltonnen bzw. Müllkästen mit einem verschließbaren Einlaß (20) zum Einführen von Abfall und einem verschließbaren Auslaß (30) zur Abgabe des Abfalls, wobei der Einlaß (20) und der Auslaß (30) nicht gleichzeitig geöffnet sind, zumindest eine steuerbare Sperre für den Einlaß (20) und/oder Auslaß (30) vorgesehen ist, eine Zahlungseinrichtung vorgesehen ist, welche die Sperre in Abhängigkeit von einem Zahlungs- oder Abrechnungsvorgang steuert, der Einlaß (20) eine erste Verschlußeinrichtung (22) umfasst, die (mechanisch) mit dem Auslaß (30) verbunden ist und einen Schwenk- und/oder Gleit- bzw. Schiebeverschluß aufweist, und der Auslaß (30) einen Schwenk- und/oder Gleit- bzw. Schiebeverschluß (32) aufweist.

## Beschreibung

Die vorliegende Erfindung betrifft eine Abfallschleuse für Mülltonnen bzw. Müllkästen.

Es sind bereits Müllklappen zum Einwerfen von Müll bekannt, die eine Öffnung aufweisen, durch die Müll eingeworfen wird. Bei den bekannten Müllklappen kann jedoch der Abfall von jedermann durch die Öffnung eingeworfen werden und somit ist eine Zuordnung des Mülls auf einzelne oder mehrere Personen eines Personenkreises, z.B. auf verschiedene Mieter eines Mietshauses, nicht möglich. In Mietshäusern werden darüberhinaus die Müllkosten anteilig nach Wohnfläche verteilt, wodurch z.B. ein einzelner Mieter in einer großen Wohnung mehr bezahlt als zwei Mieter in einer kleineren Wohnung, obwohl nachweislich zwei Mieter mehr Abfall erzeugen und entsorgen als ein einzelner. Somit ist die Müllkostenverteilung ungerecht und die von den einzelnen Mietparteien zu zahlenden Anteile entsprechen nicht der tatsächlich von ihnen erzeugten Müllmenge. Somit fehlt auch ein Anreiz zur Müllvermeidung.

Die DE-A-41 42 206 offenbart eine Eingabe-Kontrolleinrichtung für Müllbehälter, bei welcher ein Gehäuse auf einem Gestell abgestützt ist, in das ein oben offener Müllbehälter eingestellt ist. Das Gehäuse hat eine vorderseitige Tür, die nach Einwurf einer Wertmarke geöffnet werden kann, um einen Beutel auf den Boden des Gehäuses zu stellen. Nach Schließen der Tür wird der Boden von Hand oder motorisch weggezogen, so daß der Müllbeutel in den Müllbehälter fällt. Diese bekannte Eingabe-Kontrolleinrichtung hat jedoch den Nachteil, daß, falls der Benutzer, der einen Müllbeutel in die Kontroll-Eingabeeinrichtung eingegeben hat, es unterläßt, den Boden von Hand oder motorisch zu betätigen, ein nächster Benutzer, welcher eine Wertmarke einwirft und die vordere Tür öffnet, die Eingabe-Kontrolleinrichtung schon mit Müll gefüllt findet und den eigenen Müll nicht entsorgen kann. Weiterhin kann der Motor zum Antreiben des Bodens ausfallen. Es ist somit keine vorteilhaft zuverlässige und praktische Müllentsorgung möglich.

Die DE-A-39 11 971 offenbart eine Sammeleinrichtung für Sondermüll, welche geschlossene Boxen aufweist, in welche Sondermüll durch Klappen eingeführt werden kann. Die geschlossenen, vollen Boxen werden dann mittels Paletten zur Wägung und Entleerung wegbewegt, während weitere, leere Boxen an deren Stelle angeordnet werden. Diese bekannte Sammeleinrichtung ist jedoch kompliziert im Aufbau und eignet sich daher insbesondere nicht zur Entsorgung von gewöhnlichen Hausmüll.

Es ist daher Aufgabe der vorliegenden Erfindung, eine verbesserte Abfallschleuse bereitzustellen, mit welcher eine zuverlässige und einfache Müllentsorgung möglich ist, welche insbesondere betrugssicher ist und zur Entsorgung von Hausmüll geeignet ist.

Diese Aufgabe wird erfindungsgemäß durch eine Abfallschleuse gemäß Anspruch 1 gelöst. Bevorzugte Ausführungsformen der Erfindung sind Gegenstand der Unteransprüche.

Erfindungsgemäß wird ein Abfallschleuse bereitgestellt für Mülltonnen bzw. Müllkästen mit einem verschließbaren Einlaß zum Einführen von Abfall und einem verschließbaren Auslaß zur Abgabe des Abfalls, wobei der Einlaß und der Auslaß nicht gleichzeitig geöffnet sind, zumindest eine steuerbare Sperre für den Einlaß und/oder Auslaß vorgesehen ist, eine Zahlungseinrichtung vorgesehen ist, welche die Sperre in Abhängigkeit von einem Zahlungs- oder Abrechnungsvorgang steuert, der Einlaß eine erste Verschlußeinrichtung umfasst, die, insbesondere mechanisch mit dem Auslaß verbunden ist und einen Schwenk- und/oder Gleit- bzw. Schiebeverschlußaufweist, und der Auslaß einen Schwenk- und/oder Gleit- bzw. Schiebeverschluß aufweist.

Die erfindungsgemäße Abfallschleuse weist zwei Schwenk- und/oder Gleit- bzw.

Schiebeverschlußeinrichtungen auf, welche mechanisch verbunden sind. Nachdem ein Benutzer die Sperre z.B. durch einen Zahlungsvorgang entriegelt hat, kann er durch die vordere Gleitverschlußeinrichtung Müll in die Abfallschleuse eingeben. Wegen der mechanischen Kopplung der zwei Gleitverschlußeinrichtungen, betätigt er bei Betätigung der vorderen Gleitverschlußeinrichtungen gleichzeitig auch die Gleitverschlußeinrichtung des Auslaßes, wodurch ein vorangehend eingeführter Müll aus der Abfallschleuse ausgelassen wird. Somit gewährleistet die erfindungsgemäße Abfallschleuse eine zuverlässige Müllentsorgung.

Die erfindungsgemäße Abfallschleuse ist weiterhin konstruktiv sehr einfach und kann somit robust und langzeitig ausfallsicher gestaltet werden.

Mit der erfindungsgemäßen Abfallschleuse ist ein betrugssicherer Schutz vor Fremdeinwurf von Müll in die Mülltonne bzw. den Müllkasten gewährleistet.

Mit der erfindungsgemäßen Abfallschleuse ist es weiterhin vorteilhaft möglich, Müllkosten auf einen Personenkreis derart zu verteilen, daß diese Verteilung anteilig in Abhängigkeit von der tatsächlich in die Abfallschleuse eingeführten Müllmenge erfolgt. Es ist somit vorteilhaft eine gerechte Verteilung der Müllkosten gewährleistet.

Die Abfallschleuse gemäß der vorliegenden Erfindung bietet weiterhin vorteilhaft einen Anreiz zur Müllvermeidung oder Mülltrennung, da durch den Zahlungs- oder Abrechnungsvorgang jeder Einzelne für den von ihm eingeführten Müll aufkommen muß, wodurch ein Abfallbewußtsein gefördert wird. Der erfindungsgemäßen Abfallschleuse kommt deshalb in überraschender Weise eine große ökologische Bedeutung zu.

Bei einer vorteilhaften Weiterbildung der vorliegenden Erfindung ist der Einlaß mit dem Auslaß über einen Innenraum verbunden, welcher ein vorbestimmtes Volumen, insbesondere etwa 20 Liter, aufweist.

Es ist somit vorteilhaft gewährleistet, daß eine bestimmte maximale Abfall- bzw.

Müllmenge in einem Schleusvorgang in die Abfallschleuse eingeführt bzw. eingefüllt werden kann und die Abrechnung bzw. die Verteilung der Müllkosten auf einzelne Personen bzw. Parteien keinem Unsicherheitsfaktor bezüglich der eingeführten Abfallmenge unterliegt und somit gerechter ist.
Das vorbestimmte Volumen des Innenraumes, insbesondere das an sich vorteihalfte 20 Liter-Volumen, erlaubt eine Anpassung des Innenraumes an die Standardgrößen von Haus- oder Wohnungsmülleimer, wodurch eine vorteilhafte optimale Ausnutzung des in einem einzelnen Schleusvorgang zur Verfügung stehenden Innenraumes gewährleistet ist.

In einer vorteilhaften Ausführungsform der vorliegenden Erfindung ist der Auslaß mit dem Einlaß derart verbunden, daß der Auslaß geschlossen ist, wenn der Einlaß geöffnet ist.

Es wird somit in vorteilhafter Weise ein Durchschleusen von Abfall ohne Abbuchungs- bzw. Abrechnungs- bzw. Zahlungsvorgang vermieden, wodurch eine zuverlässige Abrechnung möglich und eine gerechtere Verteilung der Müllkosten gewährleistet ist.

Bei einer weiteren bevorzugten Ausführungsform der vorliegenden Erfindung umfasst der Einlaß eine erste, insbesondere vordere und eine zweite, insbesondere hintere Verschlußeinrichtung, wobei die vordere Verschlußeinrichtung mit dem Auslaß verbunden ist und die Sperre auf die hintere Verschlußeinrichtung wirkt.

Bei dieser vorteilhaften Ausführungsform ist der Einlaß durch zwei Verschlüsse gesichert, was die Betrugssicherheit vorteilhaft wesentlich verbessert. Zusätzlich ist die Verbindung bzw. Kopplung zwischen dem Einlaß und/oder Auslaß und der Sperre, d.h. der Sperrvorgang, voneinander getrennt bzw. entkoppelt, wodurch eine weitere vorteilhafte Erhöhung der Betriebs- bzw. Betrugssicherheit erzielt wird.

Bevorzugt ist die hintere Verschlußeinrichtung des Einlasses der Abfallschleuse nur dann zugänglich, wenn die vordere Verschlußvorrichtung geöffnet ist. Weiterhin bevorzugt ist die vordere Verschlußvorrichtung nicht verschließbar, wenn die hintere Verschlußeinrichtung geöffnet ist. Am bevorzugtesten weist die vordere Verschlußeinrichtung einen Schwenk- oder Gleit- bzw. Schiebeverschluß auf.

Es ist somit vorteilhaft gewährleistet, daß die Abfallschleuse in keinem Zustand eine gleichzeitige Öffnung des Einlasses und des Auslasses zulässt, wodurch ein Umgehen des Zahlungsvorganges verhindert wird.

Bei einer weiteren Ausführungsform der vorliegenden Erfindung ist die erste, insbesondere vordere Verschlußeinrichtung über zumindest ein um Umlenkrollen geführtes Drahtseil mit dem Auslaß verbunden.

Diese Verbindung bzw. Kopplung zwischen Einlaß und Auslaß ist besonders robust und erhöht somit vorteilhaft die Betriebssicherheit und Betriebsdauer der Abfallschleuse.

Gemäß einer bevorzugten Ausführungsform ist die erste Verschlußeinrichtung und der Auslaß miteinander gelenkig verbundene Verschlußelemente umfassen, wobei die Verschlußelemente länglich ausgebildet sind und an ihren Stirnseiten in Schienen geführt werden. Insbesondere können die erste Verschlußeinrichtung und der Auslaß als eine einzige aus gelenkig verbundenen Verschlußelementen gebildete Gliederkette ausgebildet sein.

In dieser bevorzugten Ausführungsform sind der Einlaß und der Auslaß vorteilhaft einfach und robust ausgestaltet und erlauben eine einfache Öffnung und Schließung des Einlasses bzw. Auslasses.

In einer weiteren bevorzugten Ausführungsform ist ein Rückstellmechanismus vorgesehen, welcher den Einlaß und/oder den Auslaß in eine ursprüngliche Stellung vorspannt.

Es ist somit vorteilhaft gewährleistet, daß die Abfallschleuse sich normalerweise, d.h. in einem unbetätigten Zustand, in einer geschlossenen Stellung befindet, wodurch der Einwurf z.B. durch unberechtigte Personen, also der Fremdeinwurf, unterbunden wird. Die Betrugssicherheit ist somit vorteilhaft erhöht.

In einer weiteren bevorzugten Weiterbildung der Erfindung ist eine Wägeeinrichtung zum Wägen des in die Abfallschleuse eingeführten Abfalls vorgesehen.

Diese Vorkehrung erlaubt eine gewichtsbezogene Abrechnung bzw. Zahlung des in die Abfallschleuse eingeführten bzw. eingeschleusten Abfalles. Die Müllkosten von einzelnen Haushalten oder Mietshäusern wird von den Gemeinden bzw. Kommunen in Volumen bzw. Volumeneinheiten berechnet, während die Gemeinden mit dem Müllentsorgungsunternehmen nach Gewicht abrechnen. Es existieren daher Bestrebungen, den anfallenden Abfall vorteilhaft schon an der Quelle, d.h. in den Haushalten, nach Gewicht abzurechnen, um eine gerechtere Abrechnung zu gewährleisten, welche die Bürger zur Müllvermeidung und/oder Mülltrennung anregt. Eine solche gewichtabhängige Müllabrechnung ist bei dieser Ausführungsform der erfindungsgemäßen Abfallschleuse vorteilhaft ermöglicht.

In einer weiterhin bevorzugten Ausführungsform der vorliegenden Erfindung ist eine Kennzeichnungseinrichtung vorgesehen, welche den in die Abfallschleuse eingeführten Abfall, insbesondere in Form von Beuteln, mit einer Kennzeichnung versieht.

Die Kennzeichnungsvorrichtung, bevorzugt in Verbindung mit der Zahlungseinrichtung, erlaubt vorteilhaft die Identifizierung der Person bzw. Mietpartei, welche den Abfall in die Abfallschleuse eingeführt hat. Falls eine Person z.B. unzulässigen Abfall in die Schleuse einführt, ist somit vorteilhaft die Möglichkeit gegeben, dies zu sanktionieren. Somit wird vorteilhaft ein ökologisch kosequentes Verhalten der einzelnen Personen herbeigeführt.

In einer weiteren bevorzugten Ausführungsform der vorliegenden Erfindung weist die Zahlungseinrichtung einen Abbuchungsspeicher zum Speichern von Abrechnungsvorgängen auf. Weiterhin bevorzugt weist die Zahlungseinrichtung eine Magnetkartenerfassungseinrichtung auf, welche am bevorzugtesten eine Abtasteinrichtung zum Abtasten von Daten einer Magnetkarte, einen Speicher und eine Vergleichseinrichtung umfasst, wobei die Vergleichseinrichtung die Daten von der Abtasteinrichtung mit jenen von dem Speicher vergleicht.

Bei diesen bevorzugten Ausführungsformen kann vorteilhaft jede einzelne Benutzung der Abfallschleuse durch Personen, welche in Besitz einer Magnetkarte sind, gespeichert werden, wobei insbesondere eine Befugniskontrolle durchgeführt wird, und zwar bevorzugt anhand von Daten, welche intern in einem Speicher abgespeichert sind.

In einer weiteren Weitergestaltung der vorliegenden Erfindung weist die Zahlungseinrichtung eine Kodeerfassungseinrichtung zum Erfassen eines Kodes auf, welche bevorzugt eine Eingabeeinheit zum Eingeben eines Kodes, einen Speicher und eine Vergleichseinrichtung umfasst, wobei die Vergleichseinrichtung den Kode der Eingabeeinheit mit in dem Speicher gespeicherten Daten vergleicht.

Bei diesen vorteilhaften Weitergestaltungen wird die Benutzung der Abfallschleuse nur denen gestattet, welche einen geeigneten Kode besitzen bzw. kennen, wodurch ein Zahlungs- bzw. Abbuchungsvorgang verursacht bzw. ausgelöst wird. Es wird somit effektiv ein Fremdeinwurf von Müll in die Abfallschleuse unterbunden, wodurch die Betrugssicherheit verbessert wird.

Bei einer weiteren bevorzugten Ausführungsform der vorliegenden Erfindung umfasst die Zahlungseinrichtung eine Münzeinwurfeinrichtung. Weiterhin bevorzugt weist die Zahlungseinrichtung eine Abzugseinrichtung und eine Lese/Schreibeinrichtung zum Einlesen bzw. Gutschreiben eines Guthabens auf, wobei die Abzugseinrichtung eine Einheit von dem Guthaben abzieht und den neuen Betrag gutschreibt.

Diese Zahlungsmodalitäten erlauben eine vorteilhafte einfache und zuverlässige Zahlung für den in die Abfallschleuse eingeführten Müll. Ein besonderer Vorteil ist darin zu sehen, daß verschiedene Zahlungssysteme bei vielen öffentlich zugänglichen Automaten benutzt werden, wodurch die einzelnen Einzelteile bzw. Komponenten, welche in der erfindungsgemäßen Abfallschleuse eingesetzt werden, in großen Mengen hergestellt und somit vorteihaft kostengünstig sind und sich durch eine hohe Betriebs- und Betrugssicherheit auszeichnen.

Gemäß einer weiteren bevorzugten AUsführungsform umfaßt die Zahlungseinrichtung eine Datenübertragungseinrichtung zur Übertragung von Daten an eine Datenerfassungseinrichtung, wobei die Datenübertragungseinrichtung insbesondere die Daten per Funk und/oder über ein Datenübertragungsnetz und/oder über das Telefonnetz und/oder über Datenspeicher überträgt, wobei die übertragenen Daten besonders bevorzugt einzelne oder mehrere folgender Daten umfassen: ein Datum, eine Uhrzeit, einen die einzelne Zahlungseinrichtung identifizierenden Kode, einen die einzelne Abfallschleuse identifizierenden Kode, einen Füllzustand der Mülltonne bzw. des Müllkastens, eine Anzahl von insgesamt getätigten Zahlungs- oder Abrechnungsvorgängen, einen Benutzerkode, eine Anzahl von Zahlungs- oder Abrechnungsvorgängen für einen vorbestimmten bzw. vorbestimmbaren Benutzerkode und/oder ein Gewicht des eingefüllten Abfalls.

Weitere Merkmale, Aufgaben und Vorteile der vorliegenden Erfindung werden aus der folgenden beispielhaften Beschreibung bevorzugter Ausführungsformen in Bezugnahme auf die Zeichnung hervorgehen.
- Fig. 1: ist eine Aufrißansicht einer Ausführungsform der erfindungsgemäßen Abfallschleuse,
- Fig. 2: ist Ansicht einer weiteren Ausführungsform der erfindungsgemäßen Abfallschleuse,
- Fig. 3: (A) ist eine Schnittansicht einer weiteren Ausführungsform der vorliegenden Erfindung mit geschlossenem Einlaß und geöffnetem Auslaß,
(B) ist eine Schnittansicht der in Fig. 3 (a) gezeigten Ausführungsform mit geöffnetem Einlaß und geschlossenem Auslaß, und
- Fig. 4: ist eine schematische Ansicht einer weiteren Ausführungsform der vorliegenden Erfindung.

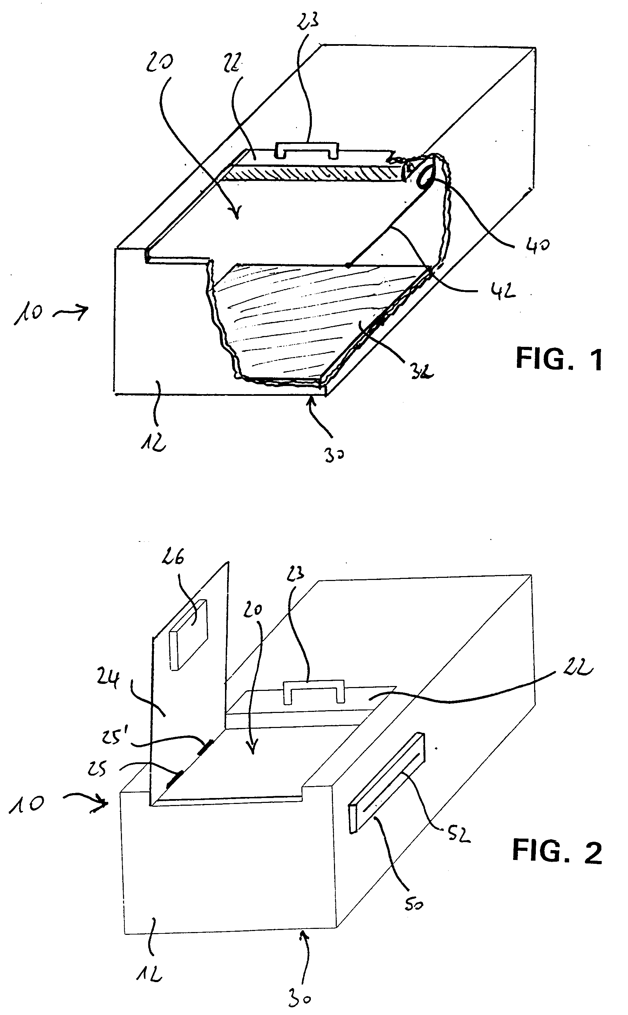

Die in Fig. 1 dargestellte Abfallschleuse 10 umfasst ein Gehäuse 12, einen verschließbaren Einlaß 20 durch welchen Müll bzw. Abfall in einen Innenraum der Abfallschleuse 10 eingeführt werden kann, und weiterhin einen Auslaß 30, durch den der Abfall von der Abfallschleuse in eine unter der Schleuse angeordneten Mülltonne bzw. Müllkasten fällt oder abgeleitet oder abgegeben wird.

Der Einlaß 20 weist einen Schiebeverschluß bzw. eine Gleitverriegelung bzw.- tür 22 auf, welche den Einlaß 20 verschließen kann. Der Schiebeverschluß 22 gleitet bevorzugt auf nicht dargestellten Schienen oder Lager und wird manuell mittels eines Griffs 23 bewegt bzw. bedient.

Der Auslaß 30 der Abfallschleuse 10 ist insbesondere an der unteren Seite der Schleuse 10 angeordnet und fluchtet insbesondere vertikal mit dem Einlaß 20. Der Auslaß 30 umfasst einen Schiebeverschluß 32, welcher die Öffnung des Auslasses 30 vollständig verschließen kann und insbesondere eine ähnliche Gestalt wie der Schiebeverschluß 22 des Einlasses aufweist.

Die Schiebeverschlüsse 22 und 32 sind miteinander verbunden bzw. gekoppelt, und zwar mittels eines über Umlenkrollen 40 geführten Drahtseils 42, welches insbesondere aus Stahl besteht, wobei die Umlenkrollen in Umfangsrichtung insbesondere Führungsnuten für das Drahtseil aufweisen. Es sind insbesondere weitere (nicht dargestellte) um Umlenkrollen geführte Drahtseile vorgesehen, um stets eine Zugkraft zwischen den miteinander verbundenen Einlaß 20 und Auslaß 30 zu übertragen. Diese (nicht dargestellten) Drahtseile sind insbesondere entlang den Seitenflächen der Abfallschleuse 10 geführt, um den Einlaß 20 und/oder den Auslaß 30 nicht zu obstruieren oder zumindest teilweise zu verschließen.
Diese Verbindung bzw. Kopplung bewirkt, daß bei einem Betätigen bzw. Bewegen insbesondere des Einlasses 20 der Auslaß 30 sich in entgegengesetzter Richtung bewegt, wodurch gewährleistet ist, daß wenn der Einlaß 20 geöffnet bzw. offen ist, der Auslaß 30 geschlossen bzw. undurchlässig ist, und wenn der Einlaß 20 geschlossen ist, der Auslaß 30 geöffnet bzw. durchlässig ist.

Die Abfallschleuse 10 weist weiterhin eine (nicht dargestellte) Zahlungseinrichtung auf, welche eine (nicht dargestellte) Sperre steuert bzw. regelt oder hemmt. Diese Sperre sperrt insbesondere den Einlaß 20 und verhindert dessen Betätigung bzw. Öffnung. Nach der Durchführung eines Abrechnungs- bzw. Abbuchungs- bzw. Zahlungsvorganges gibt die Zahlungseinrichtung ein Signal ab, welches die Sperre hemmt, und gibt somit den Einlaß 20 zur Öffnung bzw. Betätigung frei.

Die Zahlungseinrichtung umfasst einen Abbuchungsspeicher, in welchem die Abbuchungen insbesondere von verschiedenen Benutzern abgespeichert werden. Die Zahlungseinrichtung umfaßt insbesondere eine (nicht dargestellte) Kodeerfassungseinrichtung, über welche ein Benutzer der Abfallschleuse 10 einen Kennungskode zu seiner Identifizierung eingibt. Nachdem der Benutzer den Kennungskode eingegeben hat, sendet die Zahlungseinrichtung ein Signal an die Sperre, wodurch diese gehemmt wird, und ein weiteres Signal zum Abbuchungsspeicher, wodurch eine Abbuchung zu Lasten des identifizierten Benutzers stattfindet.

Fig. 2 stellt eine weitere Ausführungsform der erfindungsgemäßen Abfallschleuse 10 mit einem Gehäuse 12 dar, bei der ein Einlaß 20 zusätzlich zu dem Schiebeverschluß 22 mit Griff 23 eine Tür bzw. Klappe 24 aufweist. Der Einlaß 20 ist somit durch die Tür 24 und den Verschluß 22 verschließbar. Die Tür 24 weist insbesondere zwei Scharniere 25, 25' auf.
Eine (nicht dargestellte) steuerbare Sperre umfasst ein an der Tür 24 angebrachtes bzw. befestigtes Schloß 26, welche die Öffnung der Klappe 26 verhindern bzw. sperren bzw. blockieren kann. Die Tür 24 weist insbesondere einen (nicht dargestellten) Stift auf deren im geschlossenen Zustand zum Schiebeverschluß 22 hin gerichteten Seite auf, welcher die Öffnung des Schiebeverschlusses 22 verhindert, falls die Tür 24 nicht vollständig geschlossen ist.

Die Scharniere 25, 25' sind in einer nicht dargestellten Ausführungsform durch kreisrunde starre Scharniere ersetzt, welche in eine Seitenwand des Gehäuses 12 der Abfallschleuse 10 eingreifen. Die Sperre wirkt insbesondere auf diese Scharniere und ist insbesondere hinter einer Wandung der Seitenwand des Gehäuses angeordnet. Die Sperre stellt somit eine schwer manipulierbare Sperrung bzw. Blockierung der Tür 24 bereit und erhöht somit die Betrugssicherheit der gesamten Abfallschleuse.

Die in Fig. 2 dargestellte Abfallschleuse 10 umfasst weiterhin eine (nicht dargestellte) Magnetkartenerfassungseinrichtung mit einer Abtasteinrichtung 50 zum Abtasten einer in einem Schlitz 52 eingeführten (nicht dargestellten) Magnetkarte. Die Abtasteinrichtung 50 ist an sich bekannt und wird hier nicht näher beschrieben. Die eingeführte Magnetkarte ist insbesondere während eines Schleusvorganges in der Abtasteinrichtung 50 blockiert und wird nur am Ende des Schleusvorganges wieder freigegeben.

Auf der eingeführten Magnetkarte sind Daten gespeichert, welche durch eine (nicht dargestellte) Vergleichseinrichtung mit Daten verglichen werden, weiche in einem (nicht dargestellten) Speicher abgespeichert sind. Diese Daten enthalten insbesondere Kenndaten zum Identifizieren des Kartenbesitzers sowie eventuell auch Sicherheitsdaten, die einer Fälschung der Magnetkarte vorbeugen.

Die Abfallschleuse 10 umfasst einen Innenraum zwischen dem Einlaß 20 und dem Auslaß 30. Die Formgebung des Innenraumes ist insbesondere zylindrisch mit kreisrundem, ovalem oder rechteckigem Schnitt, und die Öffnungen des Einlasses 20 und des Auslasses 30 können insbesondere der Formgebung des Innenraumes angepaßt sein. Die Öffnung des Einlasses 20 und/oder des Auslasses 30 kann insbesondere kreisrund oder oval oder rechteckig sein.

In einer nicht dargestellten weiteren Ausführungsform der erfindungsgemäßen Abfallschleuse ist eine Wägeeinrichtung zum Wägen des in einen Innenraum der Abfallschleuse eingeführten Abfalls vorgesehen.

In einer weiteren Ausgestaltung der erfindungsgemäßen Abfallschleuse ist eine Zähleinrichtung vorgesehen, welche die Anzahl der Abbuchungsvorgänge oder die insgesamt eingeführte Abfallmenge erfasst bzw. berechnet und die Sperre in einen blockierten bzw. blockierenden Zustand versetzt` wenn eine vorbestimmte Anzahl bzw. Gesamtmenge erreicht wird.

Die Abfallschleuse der vorliegenden Erfindung ist insbesondere auf einem Müllcontainer, einer Mülltonne, einem Müllkasten oder einem Müllhäuschen oder an einem Müllschacht angebracht. Diese sind an sich bekannt und werden deshalb nicht näher beschrieben. Die Abmessungen der erfindungsgemäßen Abfallschleuse sind insbesondere an die inneren und/oder äußeren Abmessungen des Müllcontainers, der Mülltonne, des Müllkastens oder des Müllhäuschens oder des Müllschachtes angepaßt.

Ein Abfallschleusvorgang erfolgt insbesondere wie folgt:
- ein Benutzer führt eine Magnetkarte in eine Abtasteinrichtung einer Magnetkartenerfassungseinrichtung ein;
- Daten der Magnetkarte werden erfasst und durch eine Vergleichseinrichtung mit in einem Speicher gespeicherten Daten verglichen;
- falls der Vergleich "erfolgreich" ist, d.h. z.B. der Benutzer berechtigt ist, wird ein Signal zur Hemmung bzw. Unterbrechung einer Sperre und ein Signal zur Abbuchungseinrichtung übertragen;
- ein Einlaß der Abfallschleuse ist somit betätigbar bzw. kann somit geöffnet werden;
- der Benutzer öffnet den Einlaß und schließt somit gleichzeitig einen mit dem Einlaß verbundenen Auslaß der Abfallschleuse;
- der Benutzer führt bzw. füllt Abfall in einen den Einlaß und den Auslaß verbindenden Innenraum ein;
- der Benutzer schließt den Einlaß, wodurch der Auslaß geöffnet wird und der Abfall z.B. in eine unter der Abfallschleuse angeordnete Mülltonne fällt;
- der Einlaß ist wieder durch die Sperre gesperrt bzw; blockiert;
- gegebenenfalls wird ein Signal an die Abtasteinrichtung gesendet, um die darin blockierte Magnetkarte wieder freizugeben.
Die Abfallschleuse ist für einen nächsten Schleusvorgang bereit.

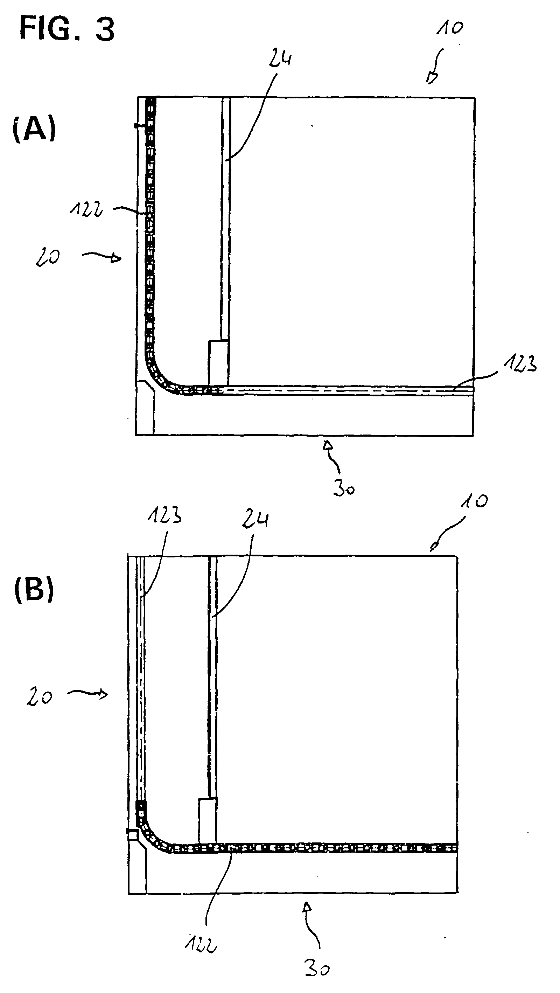

In der in Fig. 3 (A) und (B) gezeigten weiteren Ausführungsform umfassen der Einlaß 20 und der Auslaß 30 Gliederverschlüsse 122 ähnlich einem aus mehreren Gliedern bestehenden Rolladen, welche insbesondere miteinander verbunden sind. Weiterhin sind die Gliederverschlüsse insbesondere als ein einzelner, durchgehender Gliederverschluß ausgestaltet. Die Gliederverschlüsse bzw. der Gliederverschluß 122 gleiten bzw. gleitet in Nuten von Schienen 123, die insbesondere entlang der Seitenwände der Abfallschleuse angeordnet sind. In den Nuten und/oder an den Gliederverschlüssen ist eine Teflonfolie, Graphit oder dgl. angebracht, um die Gleiteingenschaften d.h. die Schmierung der Gliederverschlüsse bzw. Schienen zu verbessern.

Bei der in Fig. 3 (A) und (B) dargestellten bevorzugten Ausführungsform ist der den Gliederverschluß umfassende Einlaß 20 in einer Ebene angeordnet, welche nicht parallel zu der Ebene des Auslasses 30 ist, d.h. der Einlaß 20 ist unter einem Winkel bezüglich des Auslasses 30 angeordnet, bevorzugt ist der Einlaß 20 in einem im wesentlichen senkrechten Teil der Abfallschleuse 10 angeordnet, während der Auslaß 30 bevorzugt auf einer waagerechten unteren Seite der Abfallschleuse 10, insbesondere in Nähe des Einlasses, angeordnet ist.

In der in Fig. 3 (A) und (B) gezeigten Ausführungsform sind die erste, insbesondere vordere Verschlußeinrichtung 22 des Einlasses 20 und der Auslaß 30 sind als Gliederkette bzw. Rollo 122 ausgebildet. Die Gliederkette 122 umfaßt mitenander gelenkig bzw. schwenkbar verbundene Verschlußelemente, welche insbesondere Stabförmig ausgebildet sind und bevorzugt nur sehr kleine Zwischenräume zwischen benachbarten Verschlußelementen bilden. Die Verschlußelemente werden an ihren beiden Stirnseiten bzw. Enden in parallel, an den Seiten des Einlasses 20 und des Auslasses 30 verlaufende Schienen 123 geführt. Hinter der ersten Verschlußeinrichtung 22 ist eine zweite, insbesondere hintere Verschlußeinrichtung 24 vorgesehen. Bei Betätigung der ersten Verschlußeinrichtung 22 mittels eines (nicht gezeigten) Griffes verschieben sich die Verschlußelemente entlagen der Schienen 123, geben die zweite Verschlußeinrichtung 24 frei und verschließen gleichzeitig die Auslaß 30. Bei vollständiger Öffnung des Einlasses 20 und somit bei gleichzeitigem vollem Verschließen des Auslasses 30 ist die zweite Verschlußeinrichtung 24 betätigbar bzw. öffenbar. Somit ist ein Durchreichen von Abfall durch gleichzeitig offene erste 22 und zweite 24 Verschlußeinrichtung und Auslaß 30 nicht möglich. Bei dieser Ausführungsform weisen die Öffnungen des Einlasses 20 und des Auslasses 30 in etwa gleiche Flächen auf.

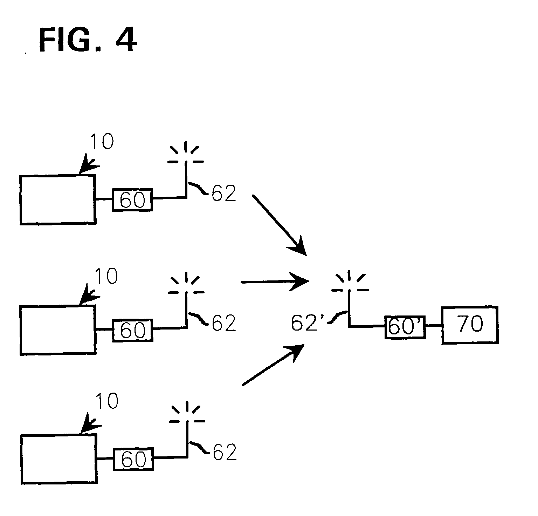

Bei der in Fig. 4 gezeigten Ausführungsform umfaßt die Abfallschleuse 10 eine Datenübertragungseinrichtung. Die Datenübertragungseinrichtung umfaßt Funkeinrichtungen 60, 60', Antennen 62, 62' und eine Auswerteinrichtung 70. Die durch die Zahlungseinrichtung erfaßten Daten können somit über Funk an eine Auswerteinrichtung übertragen werden, welche sich z.B. in einer zentralen Auswertstelle befindet. Es ist somit vorteilhaft möglich Informationen bzw. Daten von einer oder mehreren Abfallschleuse zentral an einem Ort zusammenzuführen ohne vor Ort die Daten ablesen zu müssen. Die Funkeinrichtungen 60, 60' können insbesondere an sich bekannte Funktelefone oder dgl. umfassen.

Die übertragenen Daten bzw. Informationen können insbesondere umfassen:
die Anzahl der Abbuchungsvorgänge für eine besondere Abfallschleuse,
einen ID-Kode für die Schleuse bzw. Abfalltonne, welche z.B. den Standort der Schleuse bezeichnet, die Art der Tonne betrifft usw.,
einen Füllzustand der Tonne, welcher z.B. durch die Anzahl der Füll- bzw. Schleusvorgänge, durch das vorbestimmte Volumen des Innenraums der Schleuse und durch die Größe der Mülltonne bestimmt werden kann,
ein Signal, welches angibt, daß die Mülltonne bzw- der Müllkasten voll ist, so daß die Müllabfuhr z.B. die Mülltonnen nur dann leeren muß, wenn dieses Signal gesendet wird,
ein Datum und/oder eine Uhrzeit,
eine Gesamtanzahl von getätigten Abbuchungen,
einen ID-Kode von einzelnen Benutzern und/oder eine Anzahl von Abbuchungen pro einzelnem Benutzer,
ein Gesamtgewicht von in der Mülltonne eingefülltem Müll, usw.

In nicht gezeigten Ausführungsformen kann die Datenübertragung auch über ein Datennetz (z.B. Internet), über das Telefonnetz, vor Ort über einen Datenträger (z.B. einer Diskette oder einem Magnetband) und/oder mittels einer Schnittstelle über einen (insbesondere tragbaren) Rechner.

In einer weiteren nicht gezeigten Ausführungsform ist eine Datenübertragungseinrichtung gemeinsam für mehrere, benachbart angeordnete Abfallschleusen.

## Patentansprüche

**1.** Abfallschleuse (10) für Mülltonnen bzw. Müllkästen mit einem verschließbaren Einlaß (20) zum Einführen von Abfall und einem verschließbaren Auslaß (30) zur Abgabe des Abfalls,
wobei
- der Einlaß (20) und der Auslaß (30) nicht gleichzeitig geöffnet sind,
- zumindest eine steuerbare Sperre für den Einlaß (20) und/oder Auslaß (30) vorgesehen ist,
- eine Zahlungseinrichtung vorgesehen ist, welche die Sperre in Abhängigkeit von einem Zahlungs- oder Abrechnungsvorgang steuert,
- der Einlaß (20) eine erste Verschlußeinrichtung (22) umfaßt, die mit dem Auslaß (30) verbunden ist und einen Schwenk- und/oder Gleit- bzw. Schiebeverschluß aufweist, und
- der Auslaß (30) einen Schwenk- und/oder Gleit- bzw. Schiebeverschluß (32) aufweist.

**2.** Abfallschleuse nach Anspruch 1, wobei der Einlaß (20) mit dem Auslaß (30) über einen Innenraum verbunden ist, welcher ein vorbestimmtes Volumen, insbesondere etwa 20 Liter, aufweist.

**3.** Abfallschleuse gemäß einem der vorangehenden Ansprüche, wobei der Einlaß (20) eine zweite Verschlußeinrichtung (24) umfaßt und die Sperre auf die zweite Verschlußeinrichtung (24) wirkt.

**4.** Abfallschleuse gemäß Anspruch 3, wobei die zweite Verschlußeinrichtung 24) nur dann zugänglich ist, wenn die erste Verschlußvorrichtung (22) geöffnet ist.

**5.** Abfallschleuse gemäß Anspruch 3 oder 4, wobei die erste Verschlußvorrichtung (22) nicht verschließbar ist, wenn die zweite Verschlußeinrichtung (24) geöffnet ist.

**6.** Abfallschleuse gemäß einem der Ansprüche 3 bis 5, wobei die erste Verschlußeinrichtung (22) über zumindest ein um Umlenkrollen (40) geführtes Drahtseil (42) mit dem Auslaß (30) verbunden ist.

**7.** Abfallschleuse gemäß einem der Ansprüche 3 bis 5, wobei die erste Verschlußeinrichtung (22) und der Auslaß (30) miteinander gelenkig verbundene Verschlußelemente (122) umfassen.

**8.** Abfallschleuse gemäß Anspruch 7, wobei die Verschlußelemente (122) länglich ausgebildet sind und an ihren Stirnseiten in Schienen (123) geführt werden.

**9.** Abfallschleuse gemäß einem der vorangehenden Ansprüche, wobei ein Rückstellmechanismus vorgesehen ist, welcher den Einlaß (20) und/oder den Auslaß (30) in eine ursprüngliche Stellung vorspannt.

**10.** Abfallschleuse gemäß einem der vorangehenden Ansprüche, wobei eine Wägeeinrichtung zum Wägen des in die Abfallschleuse (10) eingeführten Abfalls vorgesehen ist.

**11.** Abfallschleuse gemäß einem der vorangehenden Ansprüche, wobei eine Kennzeichnungseinrichtung vorgesehen ist, welche den in die Abfallschleuse (10) eingeführten Abfall, insbesondere in Form von Beuteln, mit einer Kennzeichnung versieht.

**12.** Abfallschleuse gemäß einem der vorangehenden Ansprüche, wobei die Zahlungseinrichtung einen Abbuchungsspeicher zum Speichern von Abrechnungsvorgängen aufweist.

**13.** Abfallschleuse gemäß einem der vorangehenden Ansprüche, wobei die Zahlungseinrichtung eine Magnetkartenerfassungseinrichtung aufweist.

**14.** Abfallschleuse gemäß Anspruch 13, wobei die Magnetkartenerfassungseinrichtung eine Abtasteinrichtung (50) zum Abtasten von Daten einer Magnetkarte, einen Speicher und eine Vergleichseinrichtung umfaßt, wobei die Vergleichseinrichtung die Daten von der Abtasteinrichtung (50) mit jenen von dem Speicher vergleicht.

**14.** Abfallschleuse gemäß einem der Ansprüche 1 bis 12, wobei die Zahlungseinrichtung eine Kodeerfassungseinrichtung zum Erfassen eines Kodes aufweist.

**15.** Abfallschleuse gemäß Anspruch 14, wobei die Kodeerfassungseinrichtung eine Eingabeeinheit zum Eingeben eines Kodes, einen Speicher und eine Vergleichseinrichtung umfaßt, wobei die Vergleichseinrichtung den Kode der Eingabeeinheit mit in dem Speicher gespeicherten Daten vergleicht.

**16.** Abfallschleuse gemäß einem der vorangehenden Ansprüche, wobei die Zahlungseinrichtung eine Münzeinwurfeinrichtung umfaßt.

**17.** Abfallschleuse gemäß einem der Ansprüche 1 bis 15, wobei die Zahlungseinrichtung eine Abzugseinrichtung und eine Lese/Schreibeinrichtung zum Einlesen bzw. Gutschreiben eines Guthabens umfaßt, wobei die Abzugseinrichtung eine Einheit von dem Guthaben abzieht und den neuen Betrag gutschreibt.

**18.** Abfallschleuse gemäß einem der vorangehenden Ansprüche, wobei die Zahlungseinrichtung eine Datenübertragungseinrichtung (60, 60', 62, 62') zur Übertragung von Daten an eine Datenerfassungseinrichtung (70) umfaßt, wobei die Datenübertragungseinrichtung (60 60', 62, 62') insbesondere die Daten per Funk und/oder über ein Datenübertragungsnetz und/oder über das Telefonnetz und/oder über Datenspeicher überträgt.

**19.** Abfallschleuse gemäß Anspruch 18, wobei die übertragenen Daten einzelne oder mehrere folgender Daten umfassen: ein Datum, eine Uhrzeit, einen die einzelne Zahlungseinrichtung identifizierenden Kode, einen die einzelne Abfallschleuse identifizierenden Kode, einen Füllzustand der Mülltonne bzw. des Müllkastens, eine Anzahl von insgesamt getätigten Zahlungs- oder Abrechnungsvorgängen, einen Benutzerkode, eine Anzahl von Zahlungs- oder Abrechnungsvorgängen für einen vorbestimmten bzw. vorbestimmbaren Benutzerkode und/oder ein Gewicht des eingefüllten Abfalls.
